# EUROPÄISCHE PATENTSCHRIFT

(11) **EP 2 030 561 B1**
(45) Veröffentlichungstag und Bekanntmachung des Hinweises auf die Patenterteilung: **26.10.2011**
(21) Anmeldenummer: 07017172.3
(22) Anmeldetag: 01.09.2007
(51) Int. Cl.: A61B 5/00, G01N 27/416

(54) **Messsystem zur Überwachung einer Analytkonzentration in vivo und Verfahren zur Erkennung einer Fehlfunktion eines derartigen Messsystems**
Sensor system for monitoring an analyte concentration in vivo and method for identifying a malfunction of such a sensor system
Système de mesure destiné à la surveillance d'une concentration d'analyte in vivo et procédé de reconnaissance d'une fonction erronée d'un tel système de mesure

(43) Veröffentlichungstag der Anmeldung: 04.03.2009
(73) Patentinhaber: Roche Diagnostics GmbH, 68305 Mannheim (DE); F.Hoffmann-La Roche AG, 4070 Basel (CH)
(72) Erfinder: Wieder, Herbert, 68259 Mannheim (DE); Marquant, Michael, 68309 Mannheim (DE)
(74) Vertreter: Twelmeier Mommer & Partner

(56) Entgegenhaltungen:
- EP-A- 0 161 673
- EP-A- 0 554 955
- GREEF R: "INSTRUMENTS FOR USE IN ELECTRODE PROCESS RESEARCH" JOURNAL OF PHYSICS E. SCIENTIFIC INSTRUMENTS, IOP PUBLISHING, BRISTOL, GB, Bd. 11, Nr. 1, 1978, Seiten 1-12, XP009026764 ISSN: 0022-3735

## Beschreibung

Die Erfindung geht aus von einem Messsystem zur Überwachung einer Analytkonzentration in vivo mit den im Oberbegriff des Anspruchs 1 angegebenen Merkmalen, wie es aus der EP 0 554 955 A1 bekannt ist.

Ein erster Teil eines derartigen Messsystems ist ein Elektrodensystem, das für eine Messung in den Körper eines Patienten implantiert oder insertiert wird. Zu dem Elektrodensystem gehören eine Arbeitselektrode, eine Referenzelektrode und eine Gegenelektrode. Als zweiter Teil gehört zu einem derartigen Messsystem ein Potentiostat, mit dem die Potentialdifferenz zwischen der Arbeitselektrode und der Referenzelektrode auf einen vorgegebenen Wert eingestellt und der Stromfluss zwischen der Arbeitselektrode und der Gegenelektrode erfasst wird. Aus der Stärke dieses Stroms kann durch Auswertung auf die gesuchte Analytkonzentration geschlossen werden.

Implantierbare oder insertierbare Elektrodensysteme ermöglichen Messungen von physiologisch bedeutsamen Analyten wie beispielsweise Glukose oder Laktat im Körper eines Patienten. Derartige Messungen in vivo haben gegenüber Vorgehensweisen, bei denen eine Probe einer Körperflüssigkeit entnommen wird und außerhalb des Körpers analysiert wird, eine Reihe wichtiger Vorteile, insbesondere die Möglichkeit einer automatischen und kontinuierlichen Erfassung von Messwerten.

Trotz dieser Vorteile konnten sich Messsysteme zur Überwachung von Analytkonzentrationen in vivo bisher am Markt nicht durchsetzen, insbesondere nicht für mobile Anwendungen, wie beispielsweise das "home-monitoring", bei dem medizinische Laien außerhalb von Krankenhäusern selbstständig ihren Blutzuckerspiegel oder eine andere Analytkonzentration überwachen. Dies liegt nicht zuletzt daran, dass sich mit bekannten tragbaren Messsystem Analytkonzentrationswerte über einen Zeitraum von mehreren Tagen nur in Ausnahmefällen mit der für medizinische Anwendungen erforderlichen Präzision und Zuverlässigkeit ermitteln lassen.

Aufgabe der Erfindung ist es deshalb, einen Weg aufzuzeigen, wie bei tragbaren Messsystemen die Zuverlässigkeit von in vivo Messdaten erhöht werden kann.

Diese Aufgabe wird durch ein Messsystem mit den im Anspruch 1 angegebenen Merkmalen gelöst. Vorteilhafte Weiterbildungen der Erfindung sind Gegenstand der Unteransprüche. Die Erfindung wird ferner durch ein Verfahren zur Erkennung einer Fehlfunktion eines Messsystems mit den im Anspruch 9 angegebenen Merkmalen gelöst.

Erfindungsgemäß wird zur Erkennung einer Fehlfunktion des Messsystems das elektrische Potential der Gegenelektrode überwacht. Anhand dieses Potentials kann eine evtl. Fehlfunktion des Messsystems frühzeitig erkannt werden und von einer Auswerteeinheit ein Fehlfunktionssignal erzeugt werden. Das Fehlfunktionssignal kann beispielsweise genutzt werden, um einen Benutzer durch ein Warnsignal auf die Fehlfunktion aufmerksam zu machen. Möglich ist es aber auch, dass das Fehlfunktionssignal systemintern verarbeitet wird und ein Benutzer allenfalls indirekt davon Kenntnis erlangt. Beispielsweise kann sich das Messsystem auf ein Fehlfunktionssignal hin vorübergehend abschalten. Auch können während einer Fehlfunktion erzeugte Messdaten auf ein Fehlfunktionssignal hin als unzuverlässig eingestuft werden, so dass eine Auswertung nicht durch während einer Fehlfunktion des Messsystems gewonnene Daten verfälscht wird.

Bei einem erfindungsgemäßen Messsystem erzeugt eine Auswerteeinheit ein Fehlfunktionssignal, wenn das elektrische Potential der Gegenelektrode außerhalb eines vorgegebenen Referenzbereichs liegt. Das elektrische Potential der Gegenelektrode wird vorzugsweise relativ zu der Arbeitselektrode gemessen. Möglich ist es aber auch das elektrische Potential der Gegenelektrode relativ zu einem anderen Bezugspotential zu messen und den Referenzbereich entsprechend festzulegen..

Der Referenzbereich des elektrischen Potentials der Gegenelektrode gibt einen Wertebereich an, in dem die bei fehlerfreiem Betrieb zu erwartenden Werte des Potentials der Gegenelektrode relativ zu einem Bezugs- oder Massepotential liegen. Werden außerhalb des Referenzbereichs liegende Werte des elektrischen Potentials der Gegenelektrode festgestellt, so deutet dies auf eine Fehlfunktion hin.

Der Referenzbereich kann für ein gegebenes Elektrodensystem fest vorgegeben werden. Bevorzugt wird der Referenzbereich, d. h. dessen Grenzen, als eine Funktion des Stromflusses zwischen der Arbeitselektrode und der Gegenelektrode festgelegt. Grenzen des Referenzbereichs können auf diese Weise von der Auswerteeinheit nach einer vorgegebenen Formel aus dem Strom berechnet werden oder einer gespeicherten Tabelle entnommen werden. Entsprechende Daten können der Auswerteeinheit auf einem Datenträger zur Verfügung gestellt werden, der zusammen mit einem den Elektroden ausgeliefert wird und beispielsweise auch Kalibrierungsdaten zur Messsensitivität enthalten kann.

Möglich ist es auch, den Referenzbereich, genauer gesagt dessen Grenzen, als eine Funktion eines früheren Wertes der elektrischen Spannung zwischen der Arbeitselektrode und der Gegenelektrode festzulegen. Schnelle Änderungen der Spannung zwischen der Arbeitselektrode und der Gegenelektrode deuten nämlich auf eine Fehlfunktion hin, da sich die Analytkonzentration in den Sensor umgebendem Körpergewebe oder Körperflüssigkeit aus physiologischen Gründen nur relativ langsam ändern kann. Sehr schnelle Änderungen können insbesondere auch in Form von Spannungsspitzen bei schlechten Kontaktstellen auftreten.

Der Referenzelektrodenanschluss und der Gegenelektrodenanschluss des Potentiostaten können über einen Sicherheitswiderstand miteinander verbunden sein. Eine fehlerhafte Messung der Potentialdifferenz zwischen der Arbeitselektrode und der Referenzelektrode, die beispielsweise durch eine schlechte Kontaktierung der Referenzelektrode verursacht ist, kann dazu führen, dass der Potentiostat übersteuert und die Elektroden zerstört. Derartigen Schäden kann ein hochohmiger den Referenzelektrodenanschluss und der Gegenelektrodenanschluss verbindender Sicherheitswiderstand vorbeugen. Bevorzugt hat der Sicherheitswiderstand einen Widerstandswert von mindestens 100 MOhm, insbesondere mindestens einem GOhm.

Wird ein derartiger Sicherheitswiderstand verwendet, wird bevorzugt zusätzlich auch die Potentialdifferenz der elektrischen Potentiale der Gegenelektrode und der Referenzelektrode überwacht. Die Erfinder haben erkannt, dass sich auch durch Auswertung dieser Spannung Fehlfunktionen des Messsystems erkennen lassen. Beispielsweise deutet eine Identität der elektrischen Potentiale des Gegenelektrodenanschluss und des Referenzelektrodenanschluss auf eine schlechte Kontaktierung der Referenzelektrode hin. Ein weiterer Aspekt der Erfindung, der auch selbstständig Bedeutung haben kann, betrifft deshalb ein Messsystem mit den im Oberbegriff des Anspruchs 1 angegebenen Merkmalen, bei dem der Referenzelektrodenanschluss und der Gegenelektrodenanschluss des Potentiostaten über einen Sicherheitswiderstand miteinander verbunden sind und eine Auswerteeinheit im Betrieb die Potentialdifferenz der elektrischen Potentiale der Gegenelektrode und der Referenzelektrode überwacht. Eine zusätzliche Überwachung des Potentials der Gegenelektrode ist zwar vorteilhaft und ermöglicht es, Fehlfunktionen mit einer größeren Zuverlässigkeit zu erkennen, jedoch können manche Fehlfunktionen bereits durch eine Überwachung der Potentialdifferenz zwischen der Gegenelektrode und der Referenzelektrode alleine erkannt werden.

Ein derartiger Sicherheitswiderstand hat jedoch den Nachteil, Kriechströme verursachen zu können. Einer Zerstörung der Elektroden wegen einer Übersteuerung des Potentiostaten kann auch ohne Sicherheitswiderstand dadurch begegnet werden, dass das elektrische Potential der Gegenelektrode ständig überwacht und das Messsystem abgeschaltet wird, sobald das Potentials außerhalb des vorgegebenen Referenzbereichs liegt.

Bevorzugt ist ein erfindungsgemäßes Messsystem mit einem Prüfwiderstand ausgestattet, der einen Selbsttest ermöglicht. Indem zu Prüfzwecken der Arbeitselektrodenanschluss des Potentiostaten über den Prüfwiderstand mit dem Gegenelektrodenanschluss des Potentiostaten verbunden wird, kann eine Sollspannung an den Prüfwiderstand angelegt werden und der durch den Prüfwiderstand fließende Strom mit einem Sollwert, der sich bei einer fehlerfreien Funktion des Potentiostaten ergibt, verglichen werden. Die Arbeitselektrode, die Gegenelektrode, die Referenzelektrode des Messsystems werden für einen solchen Selbsttest bevorzugt durch Öffnen eines mit dem betreffenden Anschluss des Potentiostaten in Reihe geschalteten Schalters von dem Potentiostaten abgekoppelt.

Da sich durch einen mit dem Prüfwiderstand durchgeführten Selbsttest Fehlfunktionen des Messsystems unabhängig von einer Auswertung des Potentials der Gegenelektrode erkennen lassen, ist ein derartiger Prüfwiderstand ein Aspekt der vorliegenden Erfindung, der auch selbständig Bedeutung haben kann. Die vorliegende Erfindung betrifft deshalb auch ein Messsystem mit den im Oberbegriff des Anspruchs 1 angegebenen Merkmalen, das durch einen Prüfwiderstand und mindestens einen mit dem Prüfwiderstand in Reihe geschalteten Schalter gekennzeichnet ist, um zu Prüfzwecken den Arbeitselektrodenanschluss über den Prüfwiderstand mit dem Gegenelektrodenanschluss und vorzugsweise auch dem Referenzelektrodenanschluss des Potentiostaten zu verbinden.

Weitere Einzelheiten und Vorteile der vorliegenden Erfindung werden anhand eines Ausführungsbeispiels unter Bezugnahme auf die beigefügte Zeichnung erläutert. Es zeigt:

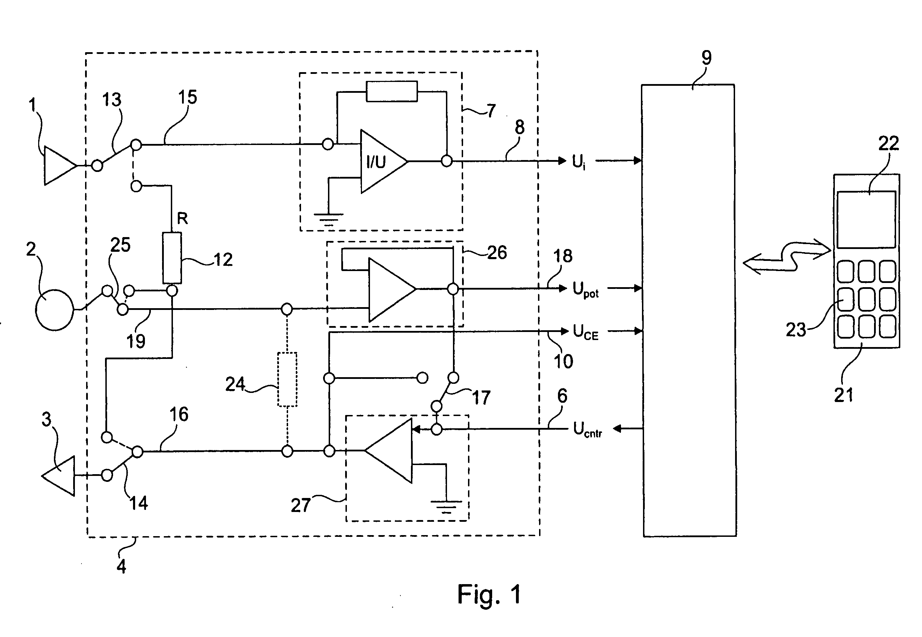

Figur 1: Eine Schaltungsskizze eines erfindungsgemäßen Messsystems.

Figur 1 zeigt eine Schaltskizze eines erfindungsgemäßen Messsystems. Ein erster Teil des Messsystems ist als Verbrauchskomponente ein implantierbares Elektrodensystem, zu dem eine Arbeitselektrode 1, eine Referenzelektrode 2 und eine Gegenelektrode 3 gehören. Die Elektroden 1, 2, 3 sind bevorzugt auf einem gemeinsamen Träger, beispielsweise einem Kunststoffblättchen, angeordnet, das bestimmungsgemäß in den Körper eines Patienten, beispielsweise das Unterhautfettgewebe, eingeführt wird.

Die Arbeitselektrode 1 trägt eine enzymhaltige Schicht mit einem Enzym, das durch katalytische Umwandlung eines Analyten Ladungsträger erzeugt, so dass ein mit der zu messenden Analytkonzentration korrelierender Strom entsteht, der zwischen der Arbeitselektrode 1 und der Gegenelektrode 3 fließt. Zur Messung der Glukosekonzentration kann beispielsweise als Enzym eine Glukoseoxidase verwendet werden.

Die Referenzelektrode 2 liefert ein Bezugspotential für die Arbeitselektrode 1, das durch eine an der Referenzelektrode ablaufende Redoxreaktion, beispielsweise Silber/Silberchlorid, definiert wird.

Das Elektrodensystem 1, 2 ,3 ist an einen Potentiostaten 4 angeschlossen, der einen zweiten Teil des Messsystems bildet. Der Potentistat 4 hat einen Arbeitelektrodenanschluss 15, an den über einen Schalter 13 die Arbeitselektrode 1 angeschlossen ist, einen Referenzelektrodenanschluss 19, an den über einen Schalter 25 die Referenzelektrode 2 angeschlossen ist, und einen Gegenelektrodenanschluss 16, an den über einen Schalter 14 die Gegenelektrode 3 angeschlossen ist.

Der Potentiostat 4 regelt im Betrieb das elektrische Potential zwischen der Arbeitselektrode 1 und der Referenzelektrode 2 auf einen vorgegebenen Wert ein, so dass durch die Referenzelektrode 2 kein oder nur ein vernachlässigbar kleiner elektrischer Strom fließt. Das gewünschte Differenzpotential wird dem Potentiostaten 4 an seinem Spannungseingang 6 als U_{cntr} vorgegeben.

Zum Regeln der elektrischen Spannung zwischen der Arbeitselektrode 1 und der Referenzelektrode 2 ist in dem Potentiostat 4, die Referenzelektrode 2 an den Eingang eines Spannungsfolgers 26 angeschlossen. Der Ausgang des Spannungsfolgers 26 liegt an dem Eingang eines Impedanzwandlers 27 an, dessen Ausgang die Spannung der Gegenelektrode 3 liefert.

Bei dem dargestellten Ausführungsbeispiel erfasst der Potentiostat 4 ferner den durch die Arbeitselektrode 1, also den zwischen der Gegenelektrode 3 und der Arbeitselektrode 1, fließenden elektrischen Strom und erzeugt mit einem Stromspannungswandler 7 ein Spannungssignal Uᵢ, das dem gemessenen Strom proportional ist und an dem Spannungsausgang 8 des Potentistaten 4 ausgegeben wird. An diesen Spannungsausgang 8 ist ein Eingang einer Auswerteeinheit 9 angeschlossen, die das Spannungssignal Uᵢ zur Bestimmung der zu messenden Analytkonzentration weiter auswertet.

Bei dem dargestellten Ausführungsbeispiel ist die Auswerteeinheit 9 als Prozessor ausgebildet und dient zugleich als Steuereinheit für den Potentiostaten 4. In der Steuer- und Auswerteeinheit 9 kann die vollständige Auswertung des Spannungssignals Uᵢ bis hin zur Bestimmung der Analytkonzentration durchgeführt werden. Bei dem dargestellten Ausführungsbeispiel erfolgt in der Auswerteeinheit 9, die von einem Patienten zusammen mit dem Potentiostaten 4 am Körper getragen wird, zunächst nur eine Vorauswertung oder eine Datenverdichtung. Die abschließende Auswertung und Berechung von Konzentrationswerten wird erst später in einem separaten Gerät 21 vorgenommen, das drahtlos mit der Auswerteeinheit 9 kommuniziert und eine Anzeigeeinrichtung 22 zum Anzeigen der gemessenen Analytkonzentrationswerte sowie Bedienungselemente 23 zum Eingeben von Steuerungsbefehlen hat.

Eine Besonderheit des dargestellten Ausführungsbeispiels besteht darin, dass das Potential der Gegenelektrode 3 über einen Spannungsausgang 10 des Potentiostaten 4 abgegriffen werden kann. Da die Arbeitselektrode 1 bei dem dargestellten Ausführungsbeispiel auf Masse liegt, stimmt das an der Gegenelektrode 3 gemessene elektrische Potential mit der elektrischen Spannung zwischen der Arbeitselektrode 1 und der Gegenelektrode 3 überein.

Im Betrieb überwacht die Auswerteeinheit 9 über den Spannungsausgang 10 des Potentiostaten 4 das elektrische Potential U_{CE} der Gegenelektrode 3 und erzeugt ein Fehlfunktionssignal, wenn dieses Potential U_{CE} außerhalb eines vorgegebenen Referenzbereichs liegt, beispielsweise um mehr als einen Schwellenwert von einem Referenzwert abweicht. Das Fehlfunktionssignal kann einem Benutzer beispielsweise visuell oder akustisch als Warnsignal übermittelt werden. Möglich ist es auch, dass das Fehlfunktionssignal ein Abschalten des Systems bewirkt, um Folgeschäden vorzubeugen. In einem solchen Fall kann vorgesehen sein, dass sich das System nach einer vorgegebenen Zeitspanne von beispielsweise 1 bis 5 Minuten, selbsttätig wieder einschaltet, da vorübergehende Fehlfunktionen beispielsweise durch unzureichenden Flüssigkeitsaustausch in der Umgebung der implantierten Elektroden 1, 2 ,3 auftreten können und sich durch Bewegungen des Patienten unter Umständen von selbst beheben können. Möglich ist es auch, dass das Messsystem bei Auftreten des Fehlfunktionssignals zunächst weiterbetrieben wird und lediglich die nach dem Auftreten des Fehlfunktionssignals gewonnenen Messergebnisse als unzuverlässig eingestuft werden. In diesem Fall ist es günstig, einen Schwellenwert zu definieren und die Auswerteeinheit 9 derart einzurichten, dass das Messsystem abgeschaltet wird, wenn das Potential U_{CE} der Gegenelektrode 3 von dem Referenzbereich um mehr als den Schwellenwert abweicht. Das Fehlfunktionssignal kann beispielsweise drahtlos an das Anzeigegerät 22 übertragen werden.

Die bei fehlerfreiem Betrieb zu erwartenden Potentialwerte hängen von dem verwendeten Sensortyp ab, beispielsweise der Größe der Elektrodenflächen und den Bedingungen der elektrochemischen Reaktion, mit der an der Arbeitselektrode 1 Ladungsträger erzeugt werden. Der Referenzbereich kann für ein gegebenes Elektrodensystem fest vorgegeben werden. Bevorzugt wird der Referenzbereich als eine Funktion des Stromflusses zwischen der Arbeitselektrode 1 und der Gegenelektrode 3 festgelegt. Der Referenzbereich können auf diese Weise von der Auswerteeinheit 8 nach einer vorgegebenen Formel aus dem aktuell gemessenen Wert des elektrischen Stroms berechnet oder einer gespeicherten Tabelle entnommen werden.

Bei dem beschriebenen Ausführungsbeispiel ist ein erster Referenzbereich als Funktion des zwischen der Arbeitselektrode 1 und der Gegenelektrode 3 fließenden Stromes festgelegt. Zum Erkennen kurzzeitiger, vorübergehender Störungen ist zusätzlich ein zweiter Referenzbereich festgelegt, dessen Grenzen jeweils eine Funktion eines früheren Werts der elektrischen Spannung zwischen der Arbeitselektrode 1 und der Gegenelektrode 3 sind.

Da sich Analytkonzentrationen in einem menschlichen Körper nur relativ langsam, in der Regel über einen Zeitraum von einigen Minuten oder Stunden, ändern, deuten starke Änderungen, die beispielsweise innerhalb von weniger als 30 Sekunden auftreten, ebenfalls auf eine Fehlfunktion hin. Mit einem Referenzbereich, der eine Funktion eines früheren Werts des elektrischen Potentials der Gegenelektrode 3 ist, können derartige Störungen schneller erkannt werden. Im einfachsten Fall kann ein vorhergehender Wert oder ein Durchschnitt einer vorgegebenen Anzahl früherer Werte als Referenzwert verwendet werden und ein dazu gehörender Schwellenwert absolut vorgegeben werden, um den Referenzbereich festzulegen, beispielsweise indem Potentialwerte, die um mehr als den Schwellenwert von dem Referenzwert abweichen, als außerhalb des Referenzbereichs definiert werden.

Die Geschwindigkeit, mit der sich Analytkonzentrationen im menschlichen Körper ändern, hängt naturgemäß von dem betreffenden Analyten, beispielsweise Glukose oder Laktat, ab, so dass keine allgemein gültigen Werte angegeben werden können. Für eine zu messende Analytkonzentration lässt sich jedoch zumindest durch Ausprobieren in der Regel ein geeigneter Referenzbereich finden, beispielsweise indem zu einem Referenzwert, der ein Funktion vorhergehender Werte sein kann, ein Schwellenwert ermittelt und als absolute Größe angegeben wird, insbesondere wenn es darum geht durch schlechte Kontakte bewirkte Spannungsspitzen als fehlerhaft zu erkennen.

Im Allgemeinen können Fehlfunktionen des beschriebenen Messsystems durch die Elektroden 1, 2, 3, schlechte Kontakte oder gar unterbrochene Leitungen oder auch durch einen Defekt des Potentiostaten 4 verursacht sein. Um bei einer festgestellten Fehlfunktion die Ursache näher eingrenzen zu können, ist das dargestellte Ausführungsbeispiel mit einem Prüfwiderstand 12 für einen Selbsttest ausgestattet.

Der Prüfwiderstand 12 ist an mindestens einem Ende an einen Schalter 13, 14, 25 angeschlossen. Über die Schalter 13, 14 kann der Prüfwiderstand 12 zu Prüfzwecken den Arbeitselektrodenanschluss 15 mit dem Gegenelektrodenanschluss 16 des Potentiostaten 4 verbinden. Über den Schalter 25 kann zudem der Referenzelektrodenanschluss 19 auf das Potential des Gegenelektrodenanschlusses 16 gelegt werden.

Für einen Selbsttest des Messsystems werden die Arbeitselektrode 1, die Referenzelektrode 2 und die Gegenelektrode 3 jeweils über die Schalter 13, 14, 25 von den betreffenden Anschlüssen 15, 16, 19 des Potentiostaten 4 abgekoppelt. Wird dann eine bekannte Spannung, beispielsweise die Sollspannung U_{cntr} der Spannungsdifferenz zwischen der Referenzelektrode 2 und der Arbeitselektrode 1, über den Schalter 17 auf den Gegenelektrodenanschluss 16 geschaltet, wird von dem System anstelle des normalerweise zwischen der Gegenelektrode 3 und der Arbeitselektrode 1 fließenden Stroms nun der durch den Prüfwiderstand 12 fließende Strom gemessen. Bei bekanntem Widerstand des Prüfwiderstands 12 kann der gemessene Strom mit einem zu erwartenden Strom verglichen werden und bei einem Vorliegen von erheblichen Abweichungen auf eine Fehlfunktion des Potentiostaten 4 geschlossen werden.

Bevorzugt ist die Auswerteeinheit 9 derart eingerichtet, dass bei einem Fehlfunktionssignal, das wegen einer auffälligen Spannung zwischen der Arbeitselektrode 1 und der Gegenelektrode 3 oder zwischen der Referenzelektrode 2 und der Gegenelektrode 3 erzeugt wurde, selbsttätig der beschriebene Selbsttest durchgeführt wird. Wird dabei eine Fehlfunktion des Potentiostaten 4 erkannt, so kann diese einem Benutzer durch ein Alarmsignal, beispielsweise ein akustisches Signal, angezeigt werden, so dass der Potentiostat 4 ausgetauscht werden kann. Wird keine Fehlfunktion des Potentiostaten 4 festgestellt, ist davon auszugehen, dass die Fehlfunktion von den Elektroden 1, 2, 3 bzw. deren Implantationsumgebung verursacht wurde. Da die Implantationsumgebung sensibel auf Bewegung des Patienten reagiert, verschwinden hierdurch verursachte Fehlfunktionen häufig von selbst, so dass ein Benutzer erst bei einer längere Zeit andauernden Fehlfunktion, die auf dem Elektrodensystem 1, 2, 3 beruht, hingewiesen werden muss.

Wird bei einem mit dem Prüfwiderstand 12 durchgeführten Selbsttest keine Fehlfunktion des Potentiostaten 4 festgestellt, führt das dargestellte Messsystem einen weiteren Selbsttest durch, bei dem die Gegenelektrode 3 durch Öffnen des Schalters 14 von dem Gegenelektrodenanschluss 16 des Potentiostaten 4 abgekoppelt ist, jedoch die Arbeitselektrode 1 und die Referenzelektrode 2 an die betreffenden Anschlüsse des Potentiostaten 4 angekoppelt sind. In diesem Fall fließt durch das Elektrodensystem 1, 2, 3 kein nennenswerter Strom mehr. Dies hat zur Folge, dass sich das elektrische Potential an der Arbeitselektrode 1 durch die dort ablaufenden elektrochemischen Prozesse ändert und einem neuen Gleichgewichtswert zustrebt.

Das stromlos an der Arbeitselektrode 1 vorliegende elektrische Potential kann als Open-Circuit-Potential bezeichnet werden. Das elektrische Potential Uₚₒₜ der Arbeitselektrode 1 wird von dem Potentiostaten 2 an seinem Spannungsausgang 18 ausgegeben und ebenfalls von der Auswerteeinheit 8 auf Auffälligkeiten überprüft. Weicht das Open-Circuit-Potential um mehr als einen vorgegebenen Schwellenwert von einem Sollwert ab, deutet dies auf eine Fehlfunktion der Arbeitselektrode 1 und/oder des Potentiostaten 4 hin. Signifikant erhöhte Werte beruhen häufig auf Veränderungen der Oberflächen der Arbeitselektrode oder der Referenzelektrode. Extrem hohe Werte deuten auf Kontaktprobleme hin.

Bei einem Potentiostaten 4 kann eine fehlerhafte Messung der Potentialdifferenz zwischen der Arbeitselektrode 1 und der Referenzelektrode 2, die beispielsweise auf einer schlechten Kontaktierung der Referenzelektrode 2 oder der Arbeitselektrode 1 beruhen kann, dazu führen, dass der Potentiostat 4 übersteuert und Schäden anrichtet, insbesondere die Elektroden zerstört. Um dem vorzubeugen, kann der Referenzelektrodenanschluss 19 über einen Sicherheitswiderstand 24 mit dem Gegenelektrodenanschluss 16 verbunden werden. Bevorzugt hat der Sicherheitswiderstand 24 einen Widerstandswert von mindestens 100 MOhm, vorzugsweise mindestens einem GOhm.

### Bezugszahlen

- 1: Arbeitselektrode
- 2: Referenzelektrode
- 3: Gegenelektrode
- 4: Potentiostat
- 7: Stromspannungswandler
- 8: Signalausgang des Potentiostaten
- 9: Auswerteeinheit
- 10: Spannungsausgang des Potentiostaten für Gegenelektrodenspannung
- 12: Prüfwiderstand
- 13: Schalter
- 14: Schalter
- 15: Arbeitselektrodenanschluss des Potentiostaten
- 16: Gegenelektrodenanschluss des Potentiostaten
- 17: Schalter
- 18: Spannungsausgang des Potentiostaten
- 19: Referenzelektrodenanschluss des Potentiostaten
- 21: Anzeigegerät
- 22: Anzeigeeinrichtung des Anzeigegeräts
- 23: Bedienungselement des Anzeigegeräts
- 24: Sicherheitswiderstand
- 25: Schalter
- 26: Spannungsfolger
- U_{CE}: Potential der Arbeitselektrode
- Uₚₒₜ: Potentialdifferenz zwischen Arbeitselektrode 1 und Referenzelektrode 2
- U_{cntr}: Sollwert der Potentialdifferenz Uₚₒₜ
- Uₗ: zu dem Strom zwischen Arbeits- und Gegenelektrode proportionales Spannungssignal

## Patentansprüche

1. Messsystem zur Überwachung einer Analytkonzentration in vivo, mit einem Elektrodensystem, das eine Arbeitselektrode (1), die eine enzymhaltige Schicht trägt, eine Referenzelektrode (2) und eine Gegenelektrode (3) aufweist, und
einem Potentiostaten (4), um eine Potentialdifferenz zwischen dem elektrischen Potential der Arbeitselektrode (1) und dem elektrischen Potential der Referenzelektrode (2) auf einen vorgegebenen Wert einzustellen und den Stromfluss zwischen der Arbeitselektrode (1) und der Gegenelektrode (3) zu erfassen, wobei der Potentiostat (4) einen Arbeitselektrodenanschluss (15) zum Anschließen an die Arbeitselektrode (1), einen Referenzelektrodenanschluss (19) zum Anschließen an die Referenzelektrode (2) und einen Gegenelektrodenanschluss (16) zum Anschließen an die Gegenelektrode (3) aufweist,
**gekennzeichnet durch**
eine Auswerteeinheit (9), welche das elektrische Potential (U_{CE}) der Gegenelektrode (3) überwacht und ein Fehlfunktionssignal erzeugt, wenn es außerhalb eines vorgegebenen Referenzbereichs liegt.

2. Messsystem nach Anspruch 1, **dadurch gekennzeichnet, dass** der Referenzbereich als eine Funktion des Stromflusses zwischen der Arbeitselektrode (1) und der Gegenelektrode (3) festgelegt wird.

3. Messsystem nach einem der vorstehenden Ansprüche, **dadurch gekennzeichnet, dass** der Referenzbereich als eine Funktion eines früheren Werts des elektrischen Potentials (U_{CE}) der Gegenelektrode (3) festgelegt wird.

4. Messsystem nach einem der vorstehenden Ansprüche, **dadurch gekennzeichnet, dass** der Referenzelektrodenanschluss (19) und der Gegenelektrodenanschluss (16) über einen Sicherheitswiderstand (24) miteinander verbunden sind.

5. Messsystem nach einem der vorstehenden Ansprüche, **gekennzeichnet durch** einen Schalter (14) zum Abkoppeln der Gegenelektrode (2) von dem Gegenelektrodenanschluss (19) des Potentiostaten (4) und/oder einen Schalter (13) zum Abkoppeln der Arbeitselektrode (1) von dem Arbeitselektrodenanschluss (15) des Potentiostaten (4).

6. Messsystem nach einem der vorstehenden Ansprüche, **gekennzeichnet durch** einen Prüfwiderstand (12) und mindestens einen mit dem Prüfwiderstand (12) in Reihe geschalteten Schalter (13), um zu Prüfzwecken den Arbeitselektrodenanschluss (15) über den Prüfwiderstand (12) mit dem Gegenelektrodenanschluss (3) des Potentiostaten (4) zu verbinden.

7. Messsystem nach Anspruch 6, **gekennzeichnet durch** einen Prüfschalter (17), um zu Prüfzwecken ein Sollpotential (U_{cntr}) an den Gegenelektrodenanschluss (16) des Potentiostaten (4) anzulegen.

8. Messsystem nach einem der vorstehenden Ansprüche, **gekennzeichnet durch** ein Anzeigegerät (22) zum Anzeigen gemessener Analytkonzentrationswerte, wobei die Auswerteeinheit (8) drahtlos mit dem Anzeigegerät (22) kommuniziert und dem Anzeigegerät (22) das Fehlfunktionssignal übermittelt, das erzeugt wird, wenn die elektrische Spannung (U_{CE}) zwischen der Arbeitselektrode (1) und der Gegenelektrode (3) um mehr als den Schwellenwert von dem Referenzwert abweicht.

9. Verfahren zur Erkennung einer Fehlfunktion eines Messsystems, das mittels eines Potentiostaten (4) und mittels eines daran angeschlossenen Elektrodensystems, das in einem Körper eines Patienten steckt, und eine Arbeitselektrode (1), die eine enzymhaltige Schicht trägt, eine Referenzelektrode (2) und eine Gegenelektrode (3) aufweist, eine Analytkonzentration in vivo misst, indem ein zwischen der Arbeitselektrode (1) und der Gegenelektrode (3) fließender elektrischer Strom gemessen wird, dessen Stärke mit der zu messenden Analytkonzentration korreliert, **dadurch gekennzeichnet, dass** zur Erkennung einer Fehlfunktion des Messsystems das elektrische Potential (U_{CE}) der Gegenelektrode (3) überwacht und ausgewertet wird.

10. Verfahren nach Anspruch 9, **dadurch gekennzeichnet, dass** zum Testen des
Messsystems die Gegenelektrode (3) von dem Potentiostaten (4) abgekoppelt und ein sich dann an der Arbeitselektrode (1) einstellendes elektrisches Potential (Uₚₒₜ) gemessen wird, wobei danach eine Abweichung des gemessenen Potentials (Uₚₒₜ) von einem Sollwert ermittelt und, falls die Abweichung einen vorgegebenen Schwellenwert übersteigt auf eine Fehlfunktion geschlossen wird.

## Claims

1. Measuring system for in vivo monitoring of an analyte concentration, having an electrode system comprising a work electrode (1) which carries an enzyme containing layer, a reference electrode (2) and a counter electrode (3) and
a potentiostat (4) for adjusting a difference of potential between the electric potential of the work electrode (1) and the electric potential of the reference electrode (2) to a specified value and for measuring an electric current flowing between the work electrode (1) and the counter electrode (3), the potentiostat (4) comprising a work electrode terminal (15) for connection to the work electrode (1), a reference electrode terminal (19) for connection to the reference electrode (2) and a counter electrode terminal (16) for connection to the counter electrode (3),
**characterized by**
an evaluation unit (9) which monitors the electric potential (U_{CE}) of the counter electrode (3) and which generates a malfunction signal when said potential is outside a specified reference range.

2. The measuring system as defined in Claim 1, **characterized in that** the reference range is specified as a function of the current flowing between the work electrode (1) and the counter electrode (3).

3. The measuring system as defined in any of the preceding claims, **characterized in that** the reference range is specified as a function of a former value of the electric potential (U_{CE}) of the counter electrode (3).

4. The measuring system as defined in any of the preceding claims, **characterized in that** the reference electrode terminal (19) and the counter electrode terminal (16) are connected one to the other via a safety resistor (24).

5. The measuring system as defined in any of the preceding claims, **characterized by** a switch (14) for decoupling the counter electrode (3) from the counter electrode terminal (19) of the potentiostat (4) and/or a switch (13) for decoupling the work electrode (1) from the work electrode terminal (15) of the potentiostat (4).

6. The measuring system as defined in any of the preceding claims, **characterized by** a testing resistor (12) and at least one switch (13) connected in series with the testing resistor (12), for connecting the work electrode terminal (15) to the counter electrode terminal (3) of the potentiostat (4) via the testing resistor (12), for testing purposes.

7. The measuring system as defined in Claim 6, **characterized by** a testing switch (17) for supplying a nominal potential (U_{cntr}) to the counter electrode terminal (16) of the potentiostat (4) for testing purposes.

8. The measuring system as defined in any of the preceding claims, **characterized by** a display (22) for displaying of measured analyte concentration values, where the evaluation unit (8) communicates with the display (22) wire-lessly for transmitting to the display (22) the malfunction signal which is produced when the electric voltage (U_{CE}) between the work electrode (1) and the counter electrode (3) deviates from the reference value by more than the threshold value.

9. Method for detecting malfunctions of a measuring system for in vivo measurement of an analyte concentration, the system comprising a potentiostat (4) and an electrode system, which is located inside a patient's body and is connected to the potentiostat, the electrode system comprising a work electrode (1) which carries an enzyme containing layer, a reference electrode (2) and a counter electrode (3), the method comprising measuring an electric current flowing between the work electrode (1) and the counter electrode (3) the intensity of which correlates with the analyte concentration to be measured, **characterized in that** the electric potential (U_{CE}) of the counter electrode (3) is monitored and evaluated for detecting malfunctions of the measuring system.

10. The method as defined in Claim 9, **characterized in that** for testing the measuring system the counter electrode (3) is decoupled from the potentiostat (4) and an electric potential (Uₚₒₜ) then occurring at the work electrode (1) is measured, whereafter any deviation of the measured potential (Uₚₒₜ) from a threshold value is detected and the existence of a malfunction is derived when the deviation exceeds a specified threshold value.

## Revendications

1. Système de mesure destiné à la surveillance d'une concentration d'analyte in vivo, comprenant un système d'électrode, lequel présente une électrode de travail (1), qui porte une couche contenant de l'enzyme, une électrode de référence (2) et une contre-électrode (3), et
un potentiostat (4), pour régler une différence de potentiel entre le potentiel électrique de l'électrode de travail (1) et le potentiel électrique de l'électrode de référence (2) sur une valeur prédéfinie et détecter le flux de courant entre l'électrode de travail (1) et la contre-électrode (3), le potentiostat (4) présentant un branchement d'électrode de travail (15) pour le raccordement à l'électrode de travail (1), un branchement d'électrode de référence (19) pour le raccordement à l'électrode de référence (2) et un raccordement de contre-électrode (16) pour le raccordement à la contre-électrode (3),
**caractérisé par**
une unité d'analyse (9), qui surveille le potentiel électrique (U_{CE}) de la contre-électrode (3) et génère un signal de dysfonctionnement lorsqu'il se situe en dehors d'une plage de référence prédéfinie.

2. Système de mesure selon la revendication 1, **caractérisé en ce que** la plage de référence est définie comme une fonction du flux de courant entre l'électrode de travail (1) et la contre-électrode (3).

3. Système de mesure selon l'une des revendications précédentes, **caractérisé en ce que** la plage de référence est définie comme une fonction d'une valeur antérieure du potentiel électrique (U_{CE}) de la contre-électrode (3).

4. Système de mesure selon l'une des revendications précédentes, **caractérisé en ce que** le raccordement d'électrode de référence (19) et le raccordement de contre-électrode (16) sont reliés l'un à l'autre par une résistance de sécurité (24).

5. Système de mesure selon l'une des revendications précédentes, **caractérisé par** un interrupteur (14) pour la dissociation de la contre-électrode (2) du raccordement de contre-électrode (19) du potentiostat (4) et/ou un interrupteur (13) pour la dissociation de l'électrode de travail (1) du raccordement d'électrode de travail (15) du potentiostat (4).

6. Système de mesure selon l'une des revendications précédentes, **caractérisé par** une résistance d'essai (12) et au moins un interrupteur (13) branché en série avec la résistance d'essai (12), afin de relier à des fins d'essai le raccordement d'électrode de travail (15) par l'intermédiaire de la résistance d'essai (12) au raccordement de contre-électrode (3) du potentiostat (4).

7. Système de mesure selon la revendication 6, **caractérisé par** un interrupteur d'essai (17), afin d'appliquer à des fins d'essai un potentiel de consigne (U_{cntr}) sur le raccordement de contre-électrode (16) du potentiostat (4).

8. Système de mesure selon l'une des revendications précédentes, **caractérisé par** un appareil d'affichage (22) pour l'affichage de valeurs mesurées de concentration d'analyte, l'unité d'analyse (8) communiquant sans fil avec l'appareil d'affichage (22) et transmettant le signal de dysfonctionnement à l'appareil d'affichage (22), lequel signal est généré lorsque la tension électrique (U_{CE}) entre l'électrode de travail (1) et la contre-électrode (3) diffère de plus de la valeur seuil de la valeur de référence.

9. Procédé destiné à détecter un dysfonctionnement d'un système de mesure, qui, au moyen d'un potentiostat (4) et au moyen d'un système d'électrode raccordé à celui-ci, lequel est enfiché dans un corps d'un patient, et présente une électrode de travail (1) portant une couche contenant de l'enzyme, une électrode de référence (2) et une contre-électrode (3), mesure une concentration d'analyte in vivo en mesurant un courant électrique circulant entre l'électrode de travail (1) et la contre-électrode (3), courant dont l'intensité est en corrélation avec la concentration d'analyte à mesurer, **caractérisé en ce que** le potentiel électrique (U_{CE}) de la contre-électrode (3) est surveillé et analysé pour identifier un dysfonctionnement du système de mesure.

10. Procédé selon la revendication 9, **caractérisé en ce que**, pour tester le système de mesure, la contre-électrode (3) est dissociée du potentiostat (4) et un potentiel électrique (Uₚₒₜ), se réglant ensuite sur l'électrode de travail (1), est mesuré, dans lequel un écart entre le potentiel (Uₚₒₜ) mesuré et une valeur de consigne est déterminé ensuite, et dans le cas où l'écart dépasse une valeur seuil prédéfinie, on conclut à un dysfonctionnement.
